# EUROPÄISCHE PATENTANMELDUNG

(11) **EP 3 020 298 A1**
(43) Veröffentlichungstag der Anmeldung: **18.05.2016**
(21) Anmeldenummer: 15193210.0
(22) Anmeldetag: 05.11.2015
(51) Int. Cl.: A43B 3/00, A43B 7/14

(54) **EINRICHTUNG ZUR DIAGNOSTIK VON DURCHBLUTUNGSSTÖRUNGEN UND SICH AUSBILDENDER ENTZÜNDUNGEN BEI FÜSSEN VON PATIENTEN MIT DIABETES**

(30) Priorität: 11.11.2014 DE 102014222955; 11.11.2014 DE 202014105408 U
(71) Anmelder: Otto-von-Guericke Universität Magdeburg Medizinische Fakultät, 39120 Magdeburg (DE)
(72) Erfinder: Mertens, Peter R., 39104 Magdeburg (DE); Klose, Silke, 39112 Magdeburg (DE)
(74) Vertreter: Kailuweit & Uhlemann Patentanwälte Partnerschaft mbB

(57) **Zusammenfassung**

Die Erfindung betrifft Einrichtungen zur Diagnostik von Durchblutungsstörungen und sich ausbildender Entzündungen bei Füssen von Patienten mit Diabetes mit Sohlen für Fussbekleidungen. Diese zeichnen sich insbesondere durch eine einfache Realisierung aus. Dazu weisen diese Sohlen für oder von Fussbekleidungen und wenigstens eine Signaleinrichtung (8) und/oder mindestens ein mit den Datenverarbeitungssystemen verbindbares und eine Datenverarbeitungseinrichtung aufweisendes externes Gerät auf.Die Sohlen umfassen dabei jeweils pro Sohle
- an bestimmten Stellen angeordnete und eingebettete Drucksensoren und erste Temperatursensoren zur Bestimmung der temperaturabhängigen Durchblutung des der Stelle zugeordneten Bereichs des jeweiligen Fusses,
- wenigstens einen zweiten Temperatursensor (4) zur Bestimmung der Umgebungstemperaturen des Fusses in der Fussbekleidung,
- mindestens entweder einen Bewegungssensor (5) oder einen Neigungsschalter,
- ein mit den Drucksensoren, den ersten Temperatursensoren, dem zweiten Temperatursensor (4) und entweder dem Bewegungssensor (5) oder dem Neigungsschalter zusammengeschaltetes Datenverarbeitungssystem (6) in Verbindung mit einem Sender/Empfänger als eine Datenübertragungseinrichtung und
- eine elektrische Energiequelle (8) in Verbindung mit wenigstens dem Datenverarbeitungssystem (6).

## Beschreibung

Die Erfindung betrifft Einrichtungen zur Diagnostik von Durchblutungsstörungen und sich ausbildender Entzündungen bei Füßen von Patienten mit Diabetes mit Sohlen für Fußbekleidungen.

Eine Folgewirkung von Diabetes ist ein Gefühlsverlust insbesondere im Fuß. Dadurch können Druckstellen entstehen, die zu Wunden führen, ohne dass dies der Patient merkt. Zur Vorbeugung und Kontrolle sind verschiedene Einrichtungen bekannt, um einer schädigenden Druckbelastung vorzubeugen.

Durch die Druckschrift DE 92 03 788 U1 ist eine Schuheinlage zur Ulcusprohylaxe am diabetischen Fuß bekannt. Dazu ist in einer Einlegesohle, welche sich ganz oder größtenteils entlang der Fußsohle erstreckt, im Bereich des Vorfußes eine kompressible Hydrozelle aus flexiblem Kunststoffmaterial angeordnet. Weiterhin steht ein Sensor zur Druckerfassung mit der Flüssigkeit in der Hydrozelle in Verbindung. Darüber hinaus ist der Sensor mit einem Zeitschalter verbunden, wobei der Sensor sowohl beim Überschreiten eines einstellbaren Maximaldruck-Schwellwerts als auch bei Erreichen eines unter diesem Maximalwert liegenden Druckschwellenwerts, sofern dieser über eine bestimmte Zeitdauer erreicht wird, ein Signal an einen Warnsignalgeber gibt. Die Druckmessung ist dabei auf einen engen Bereich der Fußsohle begrenzt. Dabei ist es schwierig, die Maximaldruckschwellenwerte und Zeitdauer festzulegen, weil sie nicht nur patientenabhängig sind, sondern auch für verschiedene Bereiche eines Fußes unterschiedlich sein können, zum Beispiel infolge von Läsionen.

Die Druckschrift US 4,647,918 A beschreibt eine Einrichtung zur Erfassung der Druckbelastung an mehreren Stellen des Fußes. Dazu wird eine Schuheinlage mit mehreren Drucksensoren verwendet. Die Drucksensoren sind an verschiedenen Stellen des Fußes platziert. Eine Signalisierung erfolgt mittels eines Geräts, welches zum einen mit den Sensoren verbunden und zum anderen durch den Patienten tragbar ist. Die Auswertung erfolgt zeitbezogen über einen Mikroprozessor, der über einen Kabelbaum elektrisch leitend mit den Messwertgebern verbunden ist, entsprechend vorgegebener und gespeicherter Grenzwerte. Eine Lichtquelle dient beispielsweise der Signalisierung. Der Kabelbaum muss dabei von der Schuheinlage über den Schuh und das Bein zum Gerät geführt werden. Dabei sind durch die Kabel hervorgerufene und nicht bestimmte Druckbelastungen am Fuß nicht auszuschließen. Insbesondere bei der Bewegung kann dieser im Schuh am Fuß reiben und zu Verletzungen führen. Das ist nur durch besondere Maßnahmen in der Fußbekleidung vermeidbar.

Durch die Druckschrift 10 2010 063 740 A1 ist eine Schutzeinrichtung für den diabetischen Fuß bekannt, die im Wesentlichen aus einer Sohle mit Seitenteilen, Sensoren als Druck- und Temperatursensoren, einem Datenverarbeitungssystem, einem Sender, einer Energiequelle, einem Empfänger und einem Signalgeber besteht. Diese Schutzeinrichtung bezieht sich insbesondere auf die Seitenteile mit Druck- und Temperaturrsensoren zur Erfassung von Eigenschaften der seitlichen Bereiche der Füße.

Die Druckschrift DE 10 2010 063 740 A1 offenbart eine Schutzeinrichtung für den diabetischen Fuß. Diese besteht aus einer Sohle mit Seitenteilen mit Druck- und Temperatursensoren für Bereiche der Fußsohle zur Messung vertikaler Belastungen und für seitliche Bereiche des Fußes zur Messung horizontaler Belastungen einschließlich der der Durchblutung zuordenbaren Temperaturen der jeweiligen Bereiche des Fußes. Die Sensorsignale werden mit einem Datenverarbeitungssystem verarbeitet. Mittels eines weiteren Temperatursensors wird die Außentemperatur erfasst. Die Messwerte werden mit Grenzwerten verglichen, wobei bei Überschreiten ein Alarm ausgelöst wird. Die Schutzeinrichtung ist auf einen Fuß beschränkt.

Die Druckschrift CHRISTIANI, Andrea M. [et al.]: An Instrumented insole for long time monitoring movement, comfort, and ergonomics. In: IEEE Sensors Journal, Vol. 14, Nr. 5, Mai 2014, S. 1564 - 1572 beinhaltet eine Sohle mit Feuchtigkeits-, Temperatur-, Druck- und Beschleunigungssensoren. Diese sind mit einem Mikroprozessor und einem Sender verbunden. Die Energieversorgung basiert auf einer Batterie. Die gewandelten und gespeicherten Messdaten können über den Sender ausgelesen werden. Die Auswertung erfolgt über einen Computer.

Die Druckschrift von SCHOENEBECK, Gudrun: Hilfe für Diabetiker - Schuheinlage mit Sensoren übernimmt das Fühlen. In: INGENIEUR.de, 17.9.2014. URL: http://www.ingenieur.de/Fachbereiche/Medizintechnik/Schuheinlage-Sensoren-uebernimmt-Fuehlen beinhaltet eine Einlegesohle zum Warnen von Diabetikern, wenn sich gefährliche Druckstellen in den Füßen entwickeln könnten. Das eingebaute Sensorsystem reagiert wie künstliche Nerven am Fuß und schickt die Messdaten aufs Smartphone des Patienten. Hauptaugenmerk gilt dem Problem des diabetischen Fußsyndroms, welches sich durch taube Füße zeigt. Die Einlegesohle besitzt ein eingearbeitetes Sensorsystem mit Temperatursensoren sowie Drucksensoren, die wie künstliche Nerven am Fuß wirken. Durch die Druckschrift US 2014/0 273 925 A1 ist ein sportliches Trainingssystem bekannt. Zur Information des Erfordernisses einer Flüssigkeits- oder Nahrungsaufnahme während des Trainings können Sensoren zum Messen von Glukose und Elektrolyt aus dem Schweiß des Benutzers vorgesehen sein.

Der im Patentanspruch 1 angegebenen Erfindung liegt die Aufgabe zugrunde, eine Einrichtung zur Diagnostik von Durchblutungsstörungen und sich ausbildender Entzündungen bei Füßen von Patienten mit Diabetes einfach zu realisieren.

Diese Aufgabe wird mit den im Patentanspruch 1 aufgeführten Merkmalen gelöst.

Die Einrichtungen zur Diagnostik von Durchblutungsstörungen und sich ausbildender Entzündungen bei Füßen von Patienten mit Diabetes mit Sohlen für oder von Fußbekleidungen zeichnen sich insbesondere durch eine einfache Realisierung aus.

Dazu weisen die Einrichtungen Sohlen für oder von Fußbekleidungen und wenigstens eine Signaleinrichtung und/oder mindestens ein mit den Datenverarbeitungssystemen verbindbares und eine Datenverarbeitungseinrichtung aufweisendes externes Gerät auf.

Die Sohlen für oder von Fußbekleidungen umfassen dabei jeweils pro Sohle
- an bestimmten Stellen angeordnete und eingebettete Drucksensoren und erste Temperatursensoren zur Bestimmung der temperaturabhängigen Durchblutung des der Stelle zugeordneten Bereichs des jeweiligen Fußes,
- wenigstens einen zweiten Temperatursensor zur Bestimmung der Umgebungstemperatur des Fußes in der Fußbekleidung,
- mindestens entweder einen Bewegungssensor oder einen Neigungsschalter,
- ein mit den Drucksensoren, den ersten Temperatursensoren, dem zweiten Temperatursensor und entweder dem Bewegungssensor oder dem Neigungsschalter zusammengeschaltetes Datenverarbeitungssystem in Verbindung mit einem Sender/Empfänger als eine Datenübertragungseinrichtung und
- eine elektrische Energiequelle in Verbindung mit wenigstens dem Datenverarbeitungssystem.

Die Datenverarbeitungssysteme der beiden Sohlen eines Patienten sind dabei
- die dem jeweiligen Fuß zugeordneten Messwerte der Drücke und Temperaturen mit einem Zeitbezug gegeneinander mittels der drahtlosen Datenübertragungseinrichtungen austauschende,
- die Messwerte der Drücke und Temperaturen in Abhängigkeit des jeweiligen Sensors miteinander und/oder mit bestimmten Grenzwerten vergleichende und jeweils der Stelle und damit dem Bereich des jeweiligen Fußes zuordnende,
- das Überschreiten eines Grenzwertes bezogen auf die Stelle und damit den Bereich zu dessen Entlastung ermittelnde,
- den mit der Ausbildung einer Entzündung einhergehenden Temperaturanstieg eines Bereichs gegenüber denselben Bereich des anderen Fußes, den Messwerten der Vortemperaturen und deren Umgebungstemperaturen ermittelnde,
- den vom Druck unabhängigen aus einer regionalen oder gesamten Minderdurchblutung hervorgerufenen Temperaturabfall eines Bereichs eines Fußes gegenüber den anderen Bereichen der Füße ermittelnde und
- die Bewegungsabläufe des Patienten erfassende Datenverarbeitungssysteme.

Sohlen für oder von Fußbekleidungen sind dazu Einlegesohlen für Fußbekleidungen oder Sohlen von Strümpfen als Fußbekleidungen oder strumpfähnlichen Fußbekleidungen.

Mittels der Einrichtung ist damit vorteilhafterweise eine
- Erkennung und Signalisierung einer lokalen Überbelastung an einer oder mehreren Stellen am Fuß,
- Früherkennung und Frühwarnung eines sich bildenden Geschwürs an einer oder mehreren Stellen am Fuß und
- Detektion eines Temperaturabfalls aufgrund einer regionalen Minderdurchblutung, die aufgrund von Gefäßverschlüssen bei Makroangiopathie zustande kommt, gegeben.

Ein Patient mit Diabetes in ihrer schweren Ausbildung hat im Fuß kein Gefühl. Deshalb besteht die Gefahr, dass er mit seinem Fuß eine Position einnimmt, die an einem oberflächlichen Ort des Fußes permanent einen Druck hervorruft. Das passiert in der Regel in Verbindung mit einer Fußbekleidung. Diesbezüglich sind vor allem die Fußsohlen gefährdet. Ein prolongierter oder permanent auf die Fußoberfläche ausgeübter Druck bewirkt eine Verringerung- oder Unterbrechung des Blutflusses in den Gefäßkapillaren, wodurch die betroffenen Gewebebereiche abkühlen. Die fehlende Durchblutung führt zu Verletzungen der betroffenen Bereiche und kann das Absterben von Gewebebereichen bewirken. Häufig wird in der Folge eine Entfernung von Zehen, zum Teil des gesamten Fußes (Amputation) unumgänglich. Bei fortbestehender Druckbelastung verheilen Wunden zudem sehr langsam. Dazu erfolgt mit der Einrichtung die Erkennung und Signalisierung einer lokalen Überbelastung an einer oder mehreren Stellen am Fuß. Mit Verwendung der Sohlen werden die lokalen Überbelastungen erfasst. Dabei werden der Druck und die Temperatur und dadurch die jeweilige Durchblutung über einen bestimmten Zeitraum gemessen und im jeweiligen Datenverarbeitungssystem gespeichert und in Intervallen oder ständig mit wenigstens einem Grenzwert verglichen. Das erfolgt für beide Füße gleichzeitig, wobei die Datenverarbeitungssysteme über die Datenübertragungseinrichtungen miteinander verbunden sind und die jeweiligen Daten austauschen. Bei intakter Durchblutung fällt die Temperatur aufgrund des Drucks bei verminderter Durchblutung ab. Wird ein Grenzwert überschritten, wird über den Sender der Datenübertragungseinrichtung ein Signal an einen Empfänger gesendet. Der Patient wird mit entsprechenden Handlungsempfehlungen, beispielsweise Ferse anheben, Ballen anheben, darauf hingewiesen, die kritische Stelle zu entlasten, um die Durchblutung wieder zu steigern. Zum Ausgleich von externen Einflüssen erfolgt die Bewertung des Temperaturprofils durch die Datenverarbeitungseinrichtung unter Einbeziehung der Messungen der zweiten Temperatursensoren zur Bestimmung der Umgebungstemperaturen der Füße in den Fußbekleidungen.

Die Früherkennung und Frühwarnung einer Entzündung und eines sich daraus bildenden Geschwürs an einer oder mehreren Stellen am Fuß kann durch Verwendung der Sohlen durch Messung eines im Falle einer entstehenden Entzündung hervorgerufenen Temperaturanstiegs erkannt werden. Vor Ausbruch eines Fußgeschwürs steigt die Temperatur etwa eine Woche im Voraus lokal an. Im Vergleich mit den Messwerten des gleichen und/oder anderen Fußes können Rückschlüsse auf ein beginnendes Ulkus gezogen werden. Wird an einem oder mehreren Sensoren ein relativer Anstieg der Temperatur mittels des Datenverarbeitungssystems erkannt und überschreitet dieser Wert einen Grenzwert, wird ein Signal über den Sender an einen Empfänger gesendet. Die Erkennung basiert dabei auf dem Vergleich der Messwerte der anderen Sensoren der gleichen Sohle und/oder der Sensoren der Sohle des zweiten Fußes. Dazu besteht eine Kommunikationsverbindung zwischen den Datenverarbeitungssystemen mittels der Datenübertragungseinrichtungen. Der Patient wird auf die Gefahr hingewiesen, dass sich ein Ulkus bildet und wird zur Selbstinspektion des Fußes auf lokale Veränderungen, Rötung, Überwärmung, Hautveränderungen aufgefordert. Gleichzeitig ist das ein Hinweis darauf, einen Arzt aufzusuchen. Gleichzeitig können die Messwerte, deren Auswertungen und die Signale als Resultate der Auswertungen an einen externen Computer gesandt werden. Daraus kann der Temperaturverlauf, das Geh- und Stehverhalten, die Nutzung der Sohle und das Entlastungsverhalten ermittelt werden. Alle Daten sind dazu zeitbezogen, das bedeutet mit der jeweils aktuellen Zeit oder einer relativen Zeit auf Basis beispielsweise eines Startsignals versehen. Ein Arzt wird somit in die Lage versetzt, mit dem Patienten Präventivmaßnahmen einzuleiten. Gleichzeitig können daraus Informationen über den technischen Zustand der Sohle abgeleitet werden.

Bei einer regionalen Minderdurchblutung in Fußbereichen oder des gesamten Fußes bei einer Durchblutungsstörung (Makroangiopathie) fällt die Temperatur ab. Die Minderdurchblutung kann akut oder chronisch eintreten, oftmals stellt sie einen wichtigen prädisponierenden Faktor für eine kritische Ischämie dar. Durch die Detektion eines druckunabhängigen Temperaturabfalls liegen Indizien für eine Ischämie vor. Dies kann dem Patienten und weiterhin dem Arzt über die Datenverarbeitungssysteme signalisiert werden. Die Sohle weist günstigerweise den Bewegungssensor auf, der mit dem Datenverarbeitungssystem zusammengeschaltet ist. Der Bewegungssensor kann ein Beschleunigungssensor oder ein Mikrosystem in Form eines Mikro-Elektro-Mechanischen-Systems (MEMS) sein. Dieses kann beispielsweise ein Feder-Masse-System mit einer Messung von Kapazitätsänderungen sein. Darüber hinaus kann auch ein Neigungsschalter mit einer Metallkugel zum Überbrücken von elektrischen Kontakten als Bewegungssensor verwendet werden. Der in der Sohle integrierte Bewegungssensor erfasst die Bewegungsabläufe des Patienten. Das kann kontinuierlich aber auch nach vorbestimmten Kriterien erfolgen. Die Messdaten können über das Datenverarbeitungssystem an einen externen Computer gesendet werden. Mit den aus den Messdaten gewonnenen Bewegungsmustern des Patienten über längere Zeiträume können Informationen über den Allgemeinzustand gewonnen werden. Dazu können bekannte Data Mining Algorithmen genutzt werden. Dadurch kann beispielsweise eine durch Fehlbelastungen verursachte Degeneration des Fußskeletts erkannt werden.

Damit ergeben sich bei Verwendung der Sohlen und damit der Einrichtung die Vorteile einer
- automatisierten Messung der regionalen Temperaturunterschiede im Fußbereich,
- Normierung der Temperaturen gegenüber der Umgebung,
- Mitteilung an den Patienten im Sinne eines Frühwarnsystems und Aufforderung zur Kontaktaufnahme mit einem Arzt oder einem Schuhmacher oder Pflegedienst,
- frühzeitigen Entlastung des Fußes und Prävention einer Geschwürsbildung,
- Frühwarnung bei Durchblutungsstörungen und
- direkten Rückmeldung und Visualisierung von beginnenden entzündlichen Prozessen im Schuh mittels bekannter Signaleinrichtungen.

Dazu können vorteilhafterweise Kombinationen jeweils aus einem Drucksensor und einem ersten Temperatursensor an verschiedenen Stellen der Sohle angeordnet sein. Damit können insbesondere die Durchblutungen des Calcaneus, des lateralen Fußbogens, des Digitus I und der Metatarsale I bis V erfasst werden.

Vorteilhafte Ausgestaltungen der Erfindung sind in den Patentansprüchen 2 bis 11 angegeben.

Der erste Temperatursensor besteht nach der Weiterbildung des Patentanspruchs 2 aus einem Schichtsystem mit einer bei Temperaturänderungen den elektrischen Widerstand ändernden Sensorschicht, die in der neutralen Faser des Schichtsystems angeordnet ist. Weiterhin ist wenigstens eine Deckschicht des Schichtsystems eine schweißresistente Deckschicht. Die Sensorschicht befindet sich auf oder in einem flexiblen Sohlenkorpus mit integrierten Kontakten zur Verbindung mit dem Datenverarbeitungssystem. Bei der Nutzung der Fußbekleidung wird der Sensor insbesondere gebogen und damit die Sensorschicht gestaucht oder gedehnt, was in Folge zu einer Widerstandsänderung führt. Diese Änderung ist aber unerwünscht, da mit dem widerstandsbasierten Sensor die Temperatur und nicht die mechanische Beanspruchung detektiert werden soll. Aus diesem Grund ist die Sensorschicht im spannungsneutralen Bereich des Schichtsystems platziert, so dass durch die Biegung hervorgerufene Messfehler minimiert sind. Die am Fuß anliegende und schweißresistente Deckschicht kann eine Deckschicht eines Temperatursensors oder eine Deckschicht aller Temperatursensoren eines Sohlenkorpusses einer Sohle sein.

Der Drucksensor ist nach der Weiterbildung des Patentanspruchs 3 günstigerweise ein piezoresistiver Drucksensor mit einer normalen Brückenschaltung, ein piezoelektrischer Drucksensor, ein kapazitiver Drucksensor oder ein Mikrosystem mit derartigen Ducksensoren. Weiterhin sind der erste Temperatursensor und der Drucksensor eine Baugruppe.

Die Sohle weist nach der Weiterbildung des Patentanspruchs 4 wenigstens einen Biosensor zur Messung des Blutzuckerspiegels über die Schweißabsonderung auf, der mit dem Datenverarbeitungssystem verbunden ist. Der Biosensor besitzt einen Rezeptor für Schweiß als Analyten, erkennt die Wechselwirkung des Analyten mit dem Rezeptor und wandelt die Veränderung des Analyten in ein elektrisches Signal. Übermäßiges Schwitzen (sogenannte Sekundäre Hyperhidrose) als Folge der Grunderkrankung Diabetes mellitus tritt bei Betroffenen nicht selten auf. Die Sekundäre Hyperhidrose tritt oft im Zusammenhang mit einer Unterzuckerung auf. Sinkt der Blutzucker des Diabetikers zu stark ab (Hypoglykämie), schüttet der Körper vermehrt Stresshormone (Adrenalin) aus. Infolgedessen kommt es zu vermehrter Schweißabsonderung. Diese verstärkte Schweißabsonderung wird bei Patienten mit Neuropathie aufgrund der fehlenden sensorischen Empfindung nicht selbstständig wahrgenommen. Mittels des Biosensors kann in diesem Fall vorteilhafterweise eine Messung des Blutzuckerspiegels erfolgen. Über das Datenverarbeitungssystem kann somit eine Signalisierung einer Unterzuckerung erfolgen. Zusätzlich kann dieser Zustand an einen externen Computer übertragen werden, wobei auch darüber eine Alarmierung des Patienten erfolgen kann.

Die Sohle besteht nach der Weiterbildung des Patentanspruchs 5 aus einem Sohlenkorpus mit
- den in Richtung des Fußes weisenden Baugruppen bestehend jeweils aus einem Drucksensor und einem ersten Temperatursensor,
- dem in Richtung vom Fuß weg weisenden zweiten Temperatursensor,
- dem Bewegungssensor oder dem Neigungsschalter,
- dem Datenverarbeitungssystem mit der Datenübertragungseinrichtung und
- der Energiequelle.

Damit ist eine kompakte Sohle vorhanden, die für den Patienten leicht handzuhaben ist.

Der Sohlenkorpus weist nach der Weiterbildung des Patentanspruchs 6 wenigstens Vertiefungen für die Baugruppen, das Datenverarbeitungssystem mit der Datenübertragungseinrichtung, die Energiequelle und entweder den Bewegungssensor oder den Neigungsschalter auf. Die Baugruppen, der zweite Temperatursensor, das Datenverarbeitungssystem mit der Datenübertragungseinrichtung, die Energiequelle und entweder der Bewegungssensor oder der Neigungsschalter sind über Kabel und/oder auf flexiblen Trägern angeordneten Leiterbahnen miteinander elektrisch leitend verbunden, wobei sich der zweite Temperatursensor entweder in der Vertiefung für das Datenverarbeitungssystem oder in einer weiteren Vertiefung befindet.

Nach der Weiterbildung des Patentanspruchs 7 ist der Sohlenkorpus günstigerweise ein aus den Daten einer Abtastung des Fußes dreidimensional realisierter Sohlenkorpus aus einem Kunststoff.

Die elektrische Energiequelle ist nach der Weiterbildung des Patentanspruchs 8 ein Akkumulator, der mit einem Steckverbinder zur lösbaren Verbindung mit einem Ladegerät verbunden ist. Damit ist der Akkumulator bei Nichtbenutzung der Sohle für eine weitere Benutzung aufladbar.

Das Datenverarbeitungssystem ist nach der Weiterbildung des Patentanspruchs 9 mit einer optische, akustische und/oder mechanische Signale abgebenden Einrichtung verbunden. Dazu können bekannte derartige signalabgebende Einrichtungen eingesetzt werden.

Der Sender der mit dem Datenverarbeitungssystem verbundenen Datenübertragungseinrichtung ist nach der Weiterbildung des Patentanspruchs 10 mit wenigstens einem Empfänger des Geräts drahtlos verbindbar.

Das Gerät ist nach der Weiterbildung des Patentanspruchs 11 ein Personalcomputer, ein mobiler Computer oder ein Mobiltelefon. Letzteres kann auch ein sogenanntes Smartphone sein.

Ein Ausführungsbeispiel der Erfindung ist in den Zeichnungen jeweils prinzipiell dargestellt und wird im Folgenden näher beschrieben.

Es zeigen:
- Fig. 1: eine Einrichtung zur Diagnostik von Durchblutungsstörungen und sich ausbildender Entzündung bei Füßen von Patienten mit Diabetes in einer Blockdarstellung,
- Fig. 2: eine Einlegesohle in einer Draufsicht und
- Fig. 3: eine Einlegesohle in einer Schnittdarstellung.

Eine Einrichtung zur Diagnostik von Durchblutungsstörungen und sich ausbildender Entzündungen bei Füßen von Patienten mit Diabetes besteht im Wesentlichen aus Sohlen für jeweils einen Fuß und wenigstens einer Signaleinrichtung 8 und/oder mindestens einem externen Gerät 11.

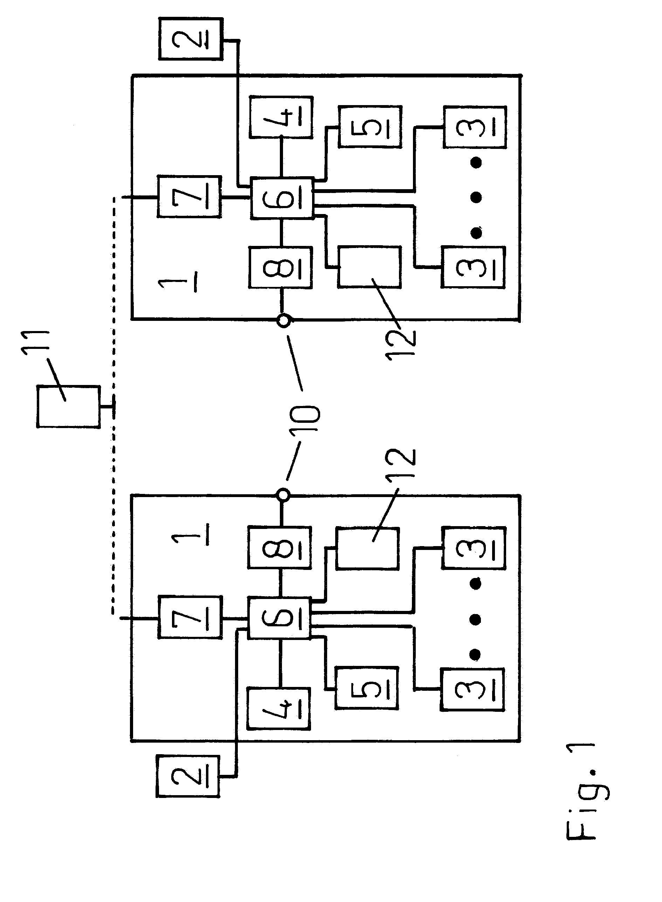

Die Fig. 1 zeigt eine Einrichtung zur Diagnostik von Durchblutungsstörungen und sich ausbildender Entzündung bei Füßen von Patienten mit Diabetes in einer prinzipiellen Blockdarstellung.

Die Sohlen sind dazu Einlegesohlen 1 für Fußbekleidungen oder Fußbekleidungen an sich, wie das beispielsweise Strümpfe oder strumpfartige Fußbekleidungen sind. Jede der Einlegesohlen 1 weist aus einem Drucksensor und einem ersten Temperatursensor bestehende Baugruppen 3, einen zweiten Temperatursensor 4, einen Bewegungssensor 5, ein Datenverarbeitungssystem 6, einen Sender/Empfänger 7 und eine elektrische Energiequelle 8 auf. Das Datenverarbeitungssystem 6 ist mit den Baugruppen 3, dem zweiten Temperatursensor 4, dem Bewegungssensor 5, dem Sender/Empfänger 7 und der elektrischen Energiequelle 8 zusammengeschaltet. Die Datenverarbeitungssysteme 6 der Einlegesohlen 1 sind über die Sender/Empfänger 7 der jeweiligen Einlegesohle 1 miteinander drahtlos verbunden. Die Sender/Empfänger 7 sind Sender/Empfänger 7 für elektromagnetische Wellen.

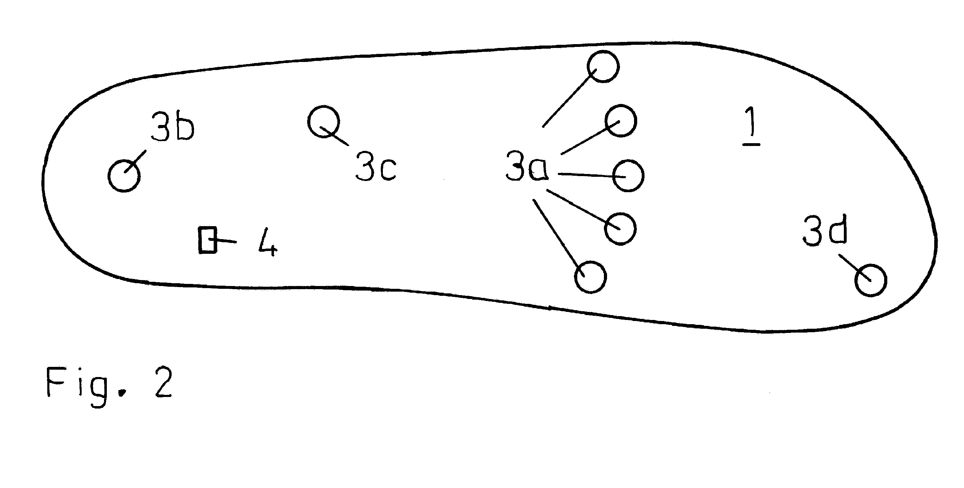

Die Fig. 2 zeigt eine Einlegesohle 1 in einer prinzipiellen Draufsicht.

Die Baugruppen 3 mit den ersten Temperatursensoren und den Drucksensoren zur Bestimmung der temperaturabhängigen Durchblutung des der Stelle zugeordneten Bereichs des jeweiligen Fußes sind an bestimmten Stellen angeordnet und in dem Sohlenkorpus der Einlegesohle 1 eingebettet. Das sind Baugruppen 3a für den Mittelfuß (Metatarsus), eine Baugruppe 3b für das Fersenbein (Calcaneus), eine Baugruppe 3d für den lateralen Fußbogen und wenigstens eine Baugruppe 3d für mindestens eine Zehe.

Der erste Temperatursensor besteht aus einem Schichtsystem mit einer bei Temperaturänderungen den elektrischen Widerstand ändernden Sensorschicht, die in der neutralen Faser des Schichtsystems angeordnet ist. Weiterhin ist wenigstens die am Fuß anliegende Deckschicht des Schichtsystems eine schweißresistente Deckschicht 9. Der erste Temperatursensor ist dazu ein bekannter widerstandsbasierter Temperatursensor, beispielsweise ein Platin-Messwiderstand oder ein Heißleiter. Letzterer ist auch als NTC-Widerstand (Negative Temperature Coefficient Thermistor) bekannt.

Der Drucksensor ist ein piezoresistiver Drucksensor, ein piezoelektrischer Drucksensor, ein kapazitiver Drucksensor oder ein einen dieser Drucksensoren aufweisendes Mikrosystem. Der erste Temperatursensor und der Drucksensor sind als ein Schichtsystem die Baugruppe 3.

Weiterhin besitzt der Sohlenkorpus der Einlegesohle 1 den Bewegungssensor 5. Das kann ein Beschleunigungssensor oder ein Mikrosystem in Form eines Mikro-Elektro-Mechanischen-Systems (MEMS) sein. Dieses kann beispielsweise ein Feder-Masse-System mit einer Messung von Kapazitätsänderungen sein. Darüber hinaus kann auch ein Neigungsschalter mit einer Metallkugel zum Überbrücken von elektrischen Kontakten als Bewegungssensor verwendet werden. Der in der Einlegesohle 5 integrierte Bewegungssensor erfasst die Bewegungsabläufe des Patienten. Das kann kontinuierlich aber auch nach vorbestimmten Kriterien erfolgen. Die Messdaten können über das Datenverarbeitungssystem 6 an das externe Gerät 11 als ein externer Computer gesendet und damit übermittelt werden. Mit den aus den Messdaten gewonnenen Bewegungsmustern des Patienten über längere Zeiträume können Informationen über den Allgemeinzustand gewonnen werden. Dazu können bekannte Data Mining Algorithmen genutzt werden. Dadurch kann beispielsweise eine durch Fehlbelastungen verursachte Degeneration des Fußskellets erkannt werden.

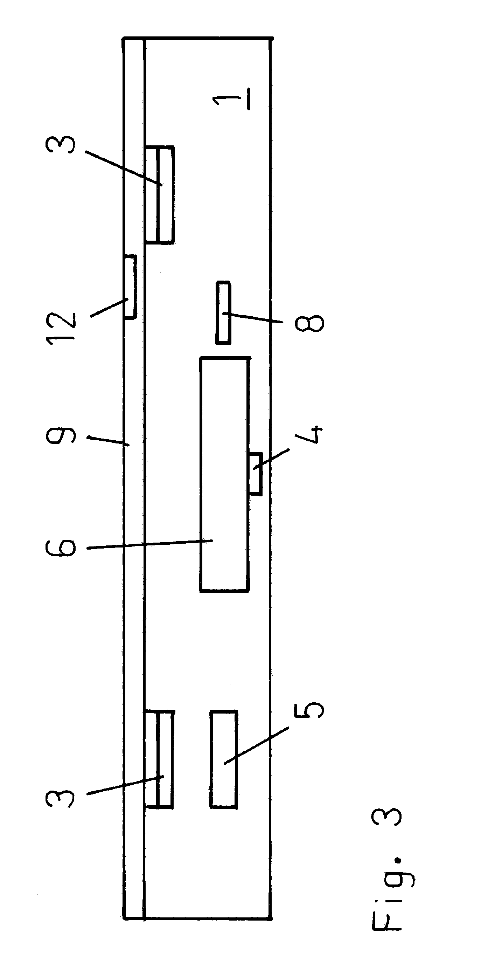

Die Fig. 3 zeigt eine Einlegesohle in einer prinzipiellen Schnittdarstellung.

Der Sohlenkorpus der Einlegesohle 1 besteht aus
- den in Richtung des Fußes weisenden Baugruppen 3 bestehend jeweils aus dem ersten Temperatursensor und dem Drucksensor,
- dem Bewegungssensor 5,
- dem in Richtung vom Fuß weg weisenden zweiten Temperatursensor 4,
- dem Datenverarbeitungssystem 6 mit dem Sender/Empfänger 7 als eine Datenübertragungseinrichtung und
- der Energiequelle 8.

Dazu weist der Sohlenkorpus Vertiefungen für die Baugruppen 3, den Bewegungssensor 5, das Datenverarbeitungssystem 6 mit der Datenübertragungseinrichtung und die Energiequelle 8 auf. Die Baugruppen 3, der Bewegungssensor 5, der zweite Temperatursensor 4, das Datenverarbeitungssystem 6 mit der Datenübertragungseinrichtung und die Energiequelle 8 sind über Kabel und/oder auf flexiblen Trägern angeordneten Leiterbahnen miteinander elektrisch leitend verbunden, wobei sich der zweite Temperatursensor 4 in der Vertiefung für das Datenverarbeitungssystem 6 befindet.

Die Datenverarbeitungssysteme 6 der beiden Einlegesohlen 1 sind
- die dem jeweiligen Fuß zugeordneten Messwerte der Drücke und Temperaturen mit einem Zeitbezug (Datum, Zeit ab Beginn der Messung, Uhrzeit) gegeneinander mittels drahtloser Datenübertragungseinrichtungen austauschende,
- die Messwerte der Drücke und Temperaturen in Abhängigkeit des jeweiligen Sensors miteinander und/oder mit bestimmten Grenzwerten vergleichende und jeweils der Stelle und damit dem Bereich des jeweiligen Fußes zuordnende,
- das Überschreiten eines Grenzwertes über die Schnittstelle bezogen auf die Stelle und damit den Bereich zu dessen Entlastung ermittelnde,
- den mit der Ausbildung einer Entzündung einhergehenden Temperaturanstieg eines Bereichs gegenüber denselben Bereich des anderen Fußes, den Messwerten der Vortemperaturen und deren Umgebungstemperaturen ermittelnde,
- den vom Druck unabhängigen aus einer regionalen oder gesamten Minderdurchblutung hervorgerufenen Temperaturabfall eines Bereichs eines Fußes gegenüber den anderen Bereichen der Füße ermittelnde und
- die Bewegungsabläufe des Patienten erfassende Datenverarbeitungssysteme 6 der Einlegesohlen 1.

Das Datenverarbeitungssystem 6 ist weiterhin mit der elektrischen Energiequelle 8 verbunden. Diese ist vorteilhafterweise ein Akkumulator, der mit einem Steckverbinder 10 zur lösbaren Verbindung mit einem Ladegerät verbunden sein kann. Der Akkumulator ist gleichfalls in einer Vertiefung des Sohlenkorpus untergebracht. Natürlich kann auch anstelle des Akkumulators eine Batterie als elektrische Energiequelle vorgesehen sein.

Das Datenverarbeitungssystem 6 kann mit einer optische, akustische und/oder mechanische Signale abgebenden Einrichtung 2 verbunden sein. Wenigstens der Sender des Sender/Empfängers 7 der mit dem Datenverarbeitungssystem 6 verbundenen Datenübertragungseinrichtung kann mit wenigstens einem Empfänger des externen Geräts 11 drahtlos verbindbar sein. Das externe Gerät 11 ist dazu vorteilhafterweise ein Computer, ein Personalcomputer, ein mobiler Computer oder ein Mobiltelefon.

Die Einlegesohle 1 weist in einer Ausführungsform weiterhin wenigstens einen Biosensor 12 zur Messung des Blutzuckerspiegels über die Schweißabsonderung auf, der mit dem Datenverarbeitungssystem 6 verbunden ist. Der Biosensor 12 besitzt einen Rezeptor für Schweiß und Glukose als Analyten, erkennt die Wechselwirkung der Analyten mit dem Rezeptor und wandelt die Veränderung derAnalyten in ein elektrisches Signal.

## Patentansprüche

1. Einrichtung zur Diagnostik von Durchblutungsstörungen und sich ausbildender Entzündungen bei Füßen von Patienten mit Diabetes mit Sohlen für oder von Fußbekleidungen mit jeweils pro Sohle
a) an bestimmten Stellen angeordneten und eingebetteten Drucksensoren und ersten Temperatursensoren zur Bestimmung der temperaturabhängigen Durchblutung des der Stelle zugeordneten Bereichs des jeweiligen Fußes,
b) wenigstens einen zweiten Temperatursensor (4) zur Bestimmung der Umgebungstemperaturen des Fußes in der Fußbekleidung,
c) mindestens entweder einen Bewegungssensor (5) oder einen Neigungsschalter,
d) einem mit den Drucksensoren, den ersten Temperatursensoren, dem zweiten Temperatursensor (4) und entweder dem Bewegungssensor (5) oder dem Neigungsschalter zusammengeschalteten Datenverarbeitungssystem (6) in Verbindung mit einem Sender/Empfänger (7) als eine Datenübertragungseinrichtung,
wobei die Datenverarbeitungssysteme (6) der beiden Sohlen für die Füße des Patienten
- die dem jeweiligen Fuß zugeordneten Messwerte der Drücke und Temperaturen mit einem Zeitbezug gegeneinander mittels der drahtlosen Datenübertragungseinrichtungen austauschende,
- die Messwerte der Drücke und Temperaturen in Abhängigkeit des jeweiligen Sensors miteinander und/oder mit bestimmten Grenzwerten vergleichende und jeweils der Stelle und damit dem Bereich des jeweiligen Fußes zuordnende,
- das Überschreiten eines Grenzwertes bezogen auf die Stelle und damit den Bereich zu dessen Entlastung ermittelnde,
- den mit der Ausbildung einer Entzündung einhergehenden Temperaturanstieg eines Bereichs gegenüber denselben Bereich des anderen Fußes, den Messwerten der Vortemperaturen und deren Umgebungstemperaturen ermittelnde,
- den vom Druck unabhängigen aus einer regionalen oder gesamten Minderdurchblutung hervorgerufenen Temperaturabfall eines Bereichs eines Fußes gegenüber den anderen Bereichen der Füße ermittelnde und
- die Bewegungsabläufe des Patienten erfassende Datenverarbeitungssysteme (6) der Sohlen sind,
e) einer elektrischen Energiequelle (8) in Verbindung mit wenigstens dem Datenverarbeitungssystem (6) und
f) wenigstens einer Signaleinrichtung (2) und/oder
g) mindestens einem mit den Datenverarbeitungssystemen (6) der Sohlen verbindbaren externen Gerät (11).

2. Einrichtung nach Patentanspruch 1, **dadurch gekennzeichnet, dass** der erste Temperatursensor aus einem Schichtsystem mit einer bei Temperaturänderungen den elektrischen Widerstand ändernden Sensorschicht besteht, dass die Sensorschicht in der neutralen Faser des Schichtsystems angeordnet ist und dass wenigstens eine am Fuß anliegende Deckschicht (9) des Schichtsystems eine schweißresistente Deckschicht (9) ist.

3. Einrichtung nach den Patentansprüchen 1 und 2, **dadurch gekennzeichnet, dass** der Drucksensor ein piezoresistiver Drucksensor, ein piezoelektrischer Drucksensor, ein kapazitiver Drucksensor oder ein einen dieser Drucksensoren aufweisendes Mikrosystem ist und dass der erste Temperatursensor und der Drucksensor eine Baugruppe (3) sind.

4. Einrichtung nach Patentanspruch 1, **dadurch gekennzeichnet, dass** die Sohle wenigstens einen Biosensor (12) zur Messung des Schweiß- und Blutzuckerspiegels über die Schweißabsonderung aufweist und dass der Biosensor (12) mit dem Datenverarbeitungssystem (6) zusammengeschaltet ist.

5. Einrichtung nach den Patentansprüchen 1 bis 3, **dadurch gekennzeichnet, dass** die Sohle aus einem Sohlenkorpus mit
- den in Richtung des Fußes weisenden Baugruppen (3) bestehend jeweils aus einem Drucksensor und einem ersten Temperatursensor,
- dem in Richtung vom Fuß weg weisenden zweiten Temperatursensor (4),
- dem Bewegungssensor (5) oder dem Neigungsschalter,
- dem Datenverarbeitungssystem (6) mit der Datenübertragungseinrichtung und
- der Energiequelle (8) besteht.

6. Einrichtung nach Patentanspruch 5, **dadurch gekennzeichnet, dass** der Sohlenkorpus wenigstens Vertiefungen für die Baugruppen (3), das Datenverarbeitungssystem (6) mit der Datenübertragungseinrichtung, die Energiequelle (8) und entweder den Bewegungssensor (5) oder den Neigungsschalter aufweist und dass die Baugruppen (3), der zweite Temperatursensor (4), das Datenverarbeitungssystem (6) mit der Datenübertragungseinrichtung, die Energiequelle (8) und entweder der Bewegungssensor (5) oder der Neigungsschalter über Kabel und/oder auf flexiblen Trägern angeordneten Leiterbahnen miteinander elektrisch leitend verbunden sind, wobei sich der zweite Temperatursensor (4) entweder in der Vertiefung für das Datenverarbeitungssystem (6) oder in einer weiteren Vertiefung befindet.

7. Einrichtung nach Patentanspruch 6, **dadurch gekennzeichnet, dass** der Sohlenkorpus ein aus den Daten einer Abtastung des Fußes dreidimensional realisierter Sohlenkorpus aus einem Kunststoff ist.

8. Einrichtung nach Patentanspruch 1, **dadurch gekennzeichnet, dass** die elektrische Energiequelle (8) ein Akkumulator ist und dass der Akkumulator mit einem Steckverbinder (10) zur lösbaren Verbindung mit einem Ladegerät verbunden ist.

9. Einrichtung nach Patentanspruch 1, **dadurch gekennzeichnet, dass** das Datenverarbeitungssystem (6) mit einer optische, akustische und/oder mechanische Signale abgebenden Einrichtung (2) verbunden ist.

10. Einrichtung nach Patentanspruch 1, **dadurch gekennzeichnet, dass** der Sender der mit dem Datenverarbeitungssystem verbundenen Datenübertragungseinrichtung mit wenigstens einem Empfänger des externen Geräts (11) drahtlos verbindbar ist.

11. Einrichtung nach Patentanspruch 10, **dadurch gekennzeichnet, dass** das externe Gerät (11) ein Personalcomputer, ein mobiler Computer oder ein Mobiltelefon ist.
